# EUROPÄISCHE PATENTSCHRIFT

(11) **EP 0 473 918 B1**
(45) Veröffentlichungstag und Bekanntmachung des Hinweises auf die Patenterteilung: **31.07.1996**
(21) Anmeldenummer: 91112009.5
(22) Anmeldetag: 18.07.1991
(51) Int. Cl.: A61F 13/02

(54) **Verbandmaterial auf Folienbasis**
Film-basis dressing
Pansement à base de film

(30) Priorität: 24.08.1990 DE 4026755
(43) Veröffentlichungstag der Anmeldung: 11.03.1992
(73) Patentinhaber: Beiersdorf Aktiengesellschaft, D-20245 Hamburg (DE)
(72) Erfinder: Schulte, Dietrich, Dr., W-2080 Pinneberg (DE); Götz, Gabriela, W-2000 Hamburg 65 (DE); Eggers, Ursula, W-2000 Hamburg 52 (DE); Ahrens, Helge, W-2000 Hamburg 60 (DE)

(56) Entgegenhaltungen:
- EP-A- 0 168 174
- EP-A- 0 254 493
- WO-A-90/01915
- US-A- 4 600 001

## Beschreibung

Die Erfindung betrifft ein Verbandmaterial auf Folienbasis gemäß Oberbegriff des Anspruchs 1.

Anschmiegsame, relativ dünne Klebematerialien aus Folien in Bahn- oder Blattform eignen sich bei der medizinischen Versorgung in vielfältiger Weise, z.B. als Inzisionsfolie zum keimfreien Abdecken der Operationsstelle, zum großflächigen Abdecken von Verbänden oder zum Fixieren von Kanülen, Kathetern und dgl.. Aufgrund ihrer Flexibilität schmiegen sie sich den Unebenheiten an und ihre meist gut ausgeprägte Luft- bzw. Wasserdampfdurchlässigkeit ermöglicht die Hautatmung.

Ein Problem stellt dabei jedoch immer die Handhabung des dünnen, lappigen Materials dar, die in der Regel nicht ohne spezielle Anfasser möglich ist. Für die Ausbildung dieser Anfaßleisten oder -ränder sind schon die verschiedensten Vorschläge gemacht und z.T. auch verwirklicht worden (vgl. z.B. EP-A 66 899, EP-B 81 987, EP-B 81 989, EP-A 117 632, EP-A 161 865, DE-A 33 17 929). Sie erfordern jedoch immer ein Abreißen oder Abschneiden des Randes bzw. weisen einen komplizierten und nur mit hohen Kosten herstellbaren Laminataufbau auf.

Um die Handhabbarkeit derartiger, vorzugsweise aus Polyurethan bestehender Folien zu verbessern, ist auch schon vorgeschlagen worden, sie auf einem Trägermaterial anzuordnen, das nachträglich wieder entfernt werden kann (vgl. z.B. EP-B 51 935, EP-B 81 990, EP-B 144 891, EP-A 168 174, WO 90 01 915, WO 89 11 262) und z.T. eine Anfaßmöglichkeit bietet.

Diese bekannten Ausführungsformen haben aber ebenfalls den Nachteil, daß sie kompliziert und erklärungsbedürftig in der Anwendung oder teuer und umständlich in der Herstellung sind.

In der EP-A 168 174 wird ein Folienverband beschrieben mit einer relativ festen zweiteiligen Trägerschicht, auf welcher die Folie durch eine Klebschicht befestigt ist. Das hat den Nachteil, daß bei nicht ganz exaktem Ankleben des Filmes dieser beim Abziehen der Trägerschicht wegen der Adhäsion der Klebeschicht leicht wieder von der Haut mit abgehoben wird. Außerdem muß die zweiteilige Trägerschicht mit beiden Händen entfernt werden.

Auch bei dem Verbandmaterial gemäß der WO 90 01 915 ist die tragende Filmschicht, die an den Seiten über die Verbandfolie hinausragt, mit dieser klebend verbunden, so daß beim Abziehen der Trägerschicht besondere Sorgfalt aufgewendet werden muß, um ein Abheben der Verbandfolie von der Haut zu verhindern.

Bei dem Folienverband gemäß der EP-B 144 891 ragt die Stützfolie ebenfalls über die Verbandfolie hinaus und die Haftung zwischen den beiden Folien muß so groß sein, daß die Kraft zum Abziehen der Abdeckung auf der Klebeschicht geringer ist als die zum Abziehen der Stützfolie von der Verbandfolie. Also auch hier besteht eine relativ starke Haftung der beiden Folien aufeinander. Die Herstellung der Anfaßränder erfolgt in der Weise, daß durch das Laminat aus Stützfolie, Verbandfolie, Klebeschicht und Abdeckung von der Abdeckungsseite her eine tiefenkontrollierte Stanzung vorgenommen wird bis zur Stützfolie und dann der Laminatteil aus Verbandfolie, Klebeschicht und Abdeckung abgezogen und verworfen wird. Insgesamt also ein materialaufwendiges und teures Verfahren.

Die EP-A 254 493 betrifft einen Wundverband, dessen Mittelteil nichtklebend ausgebildet und vorzugsweise mit einer saugfähigen Schicht hinterlegt ist und dessen Seitenteile aus klebenden Streifen bestehen, um den Verband auf der Haut zu befestigen.

In der dem Oberbegriff des Anspruchs 1 zugrundeliegenden WO 89 11 262 wird ein selbstklebendes Folienmaterial beschrieben, das aus einer Folie besteht, die auf einer Oberfläche mit einer Selbstklebeschicht versehen ist, welche mit einem entfernbaren Abziehbelag (Schutzabdeckung) bedeckt ist, und auf der nichtklebenden Oberfläche eine Stützschicht (Trägerschicht) aufweist. Gekennzeichnet ist das Produkt dadurch, daß die Abziehkraft, die benötigt wird, um den Abziehbelag von der Klebschicht zu trennen, großer ist als die Abziehkraft, die benötigt wird, um die Stützschicht von der Folie zu trennen, und der Abziehbelag steifer ist, als die Stützschicht, wodurch ein Abziehen des Abziehbelags ermöglicht wird, ohne daß die Stützschicht verschoben wird.

Die Stützschicht ist mit der Folie deckungsgleich und weist keine überstehenden Anfasser oder Griffleisten auf. Sie ist mehrteilig, mit mehreren Schnitten hergestellt und muß dann auch in mehreren Schritten entfernt werden. Die Stützschicht ist in der Regel in einen mittleren Teil und einen Randteil, der seinerseits noch wieder aufgeteilt sein kann, unterteilt.

Dies bedeutet, daß der Verband einerseits nicht so bequem aufgebracht werden kann, da kein breiter abstehender Rand zum Anfassen vorhanden ist, und andererseits auch die Stützschicht nicht mit einer einfachen Abziehbewegung entfernt werden kann.

Aufgabe der Erfindung war es demgegenüber, ein einfach handhabbares und auch unkompliziert und damit kostengünstig herstellbares Aufbringungssystem für dünne und schmiegsame Klebematerialien auf Folienbasis zu entwickeln.

Gelöst wird dieses Problem in überraschend einfacher aber effektiver Weise durch ein Verbandmaterial auf Folienbasis gemäß Anspruch 1.

Die Trägerschicht, die eine stützende Wirkung für die Folie hat und beim Aufbringen des Verbandes auf dieser verbleibt, besteht vorzugsweise aus einer Polyethylenfolie von etwa 80 Mikrometer Dicke, die auf ihrer Seite zur Folie hin leicht gerauht ist und dadurch matt, aber noch durchscheinend wirkt.

Es lassen sich auch andere Folien aus beispielsweise Polypropylen, Polyester oder PVC verwenden, sofern sie nur schmiegsam genug sind, um beim Anbringen des Verbandes nicht zu stören.

Ihre technischen Daten können sich in folgenden
Bereichen bewegen:
Dicke: 30 - 300 Mikrometer
Gewicht: 30 - 350 g/m²
Höchstzugkraft längs: 5 - 100 N/cm
Reißdehnung längs: 10 - 1000%
Ihre Oberfläche zur Folie kann glatt , gerauht oder leicht geprägt sein.

Die Griffleisten, die, in Längsrichtung des Verbandes gesehen, rechts und links außen auf den Rändern des Trägermaterials etwa 3cm breit und davon etwa 2 cm überstehend angebracht sind, bestehen vorzugsweise aus einer LDPE-Folie (low density polyethylene-Folie) von ebenfalls 80 Mikrometer Dicke. Um gut sichtbar zu sein, sind sie beispielsweise blau eingefärbt und wegen der besseren Griffigkeit an der Außenseite matt geprägt.

Anstelle dieser Folie können auch andere Materialien wie HDPE-, Polypropylen-, PVC-, PU- oder Polyesterfolien sowie Vliese, Papier oder Gewebe verwendet werden, sofern sie nur wieder ausreichend flexibel und anschmiegsam sind.

Die Dicke kann sich je nach Material zwischen 10 und 300 Mikrometer, das Gewicht entsprechend zwischen 10 bis 350 g/m² bewegen. Die Oberfläche kann matt, glänzend, rauh, glatt oder bedruckt sein. Die Höchstzugkraft längs kann je nach Material zwischen etwa 3 und 100 N/cm schwanken und die dabei auftretende Dehnung kann sich zwischen 5 und 500% bewegen.

Die Befestigung der Griffleisten an der Trägerschicht kann je nach Material und Verarbeitungsmaschinen in verschiedenster Weise erfolgen, vorzugsweise durch Kleben oder Verschweißen. Das Kleben erfolgt dabei beispielsweise in der Weise, daß zwischen der Trägerschicht und den Griffleistenstreifen im Überlappungsbereich ein Streifen eines doppelseitig klebenden Klebebandes eingebracht wird oder eine Klebemassenbeschichtung aus einer Hotmelt-Masse oder einer Selbstklebemasse aus Lösungsmittel oder Dispersion.

Die Verbandfolie selbst besteht vorzugsweise aus elastischen, thermoplastischen Polyurethanen, wie sie in der DE-C 19 34 710 beschrieben sind und die sich durch eine gute Hautverträglichkeit sowie Sauerstoff- und Wasserdampfdurchlässigkeit auszeichnen. Besonders vorteilhaft haben sich aliphatische Polyesterurethane erwiesen.

Eine bevorzugte Folie ist etwa 30 - 40 Mikrometer stark, transparent, weist eine Reißdehnung von über 450% und eine Wasserdampfdurchlässigkeit von über 500 g/m² in 24h bei 38°C und 95% rel. Feuchte nach DAB auf.

Daneben lassen sich jedoch auch Filme auf anderer Grundlage, wie z.B. Acrylat-Copolymere oder die anderen bekannten filmbildenden elastischen Polymeren, verwenden. Die Dicke der Folien kann dabei zwischen etwa 30 - 300 Mikrometer, das Gewicht entsprechend zwischen etwa 30 - 350 g/m², die Höchstzugkraft längs zwischen etwa 5 - 100 N/cm und die Reißdehnung längs zwischen etwa 10 - 1000% schwanken.

Die Haftung der Folie auf der Trägerschicht, die erfindungsgemäß gering sein muß (0,01 - 0,5 N/cm, vorzugsweise 0,01 - 0,05 N/cm), wird dadurch bewirkt, daß die dünne Folie direkt auf dem Träger erzeugt wird durch Gießen, Rakeln, Extrudieren oder andere bekannte Methoden zur Filmherstellung. Falls notwendig, kann die Trägerschicht auf der Beschichtungsseite aufgerauht oder einer anderen haftungsfördernden Behandlung unterworfen werden.

Wichtig dabei ist, daß die Haftung des fertigen Verbandes auf der Haut wesentlich stärker ist als die Haftung der Trägerschicht an der Folie.

Die Klebschicht auf der Folie weist beispielsweise bevorzugt eine Klebkraft auf Stahl von etwa 2 - 4 N/cm auf, wobei das Prüfmaterial, da die Folie sehr dehnbar ist, für die Messung rückseitig mit einem unelastischen Klebefilm verstärkt werden muß. Die Messung selbst erfolgte in Anlehnung an DAB 9.

Auf seiner selbstklebend ausgerüsteten, später der Haut zugewandten Seite ist das erfindungsgemäße Verbandmaterial über seine ganze Breite bis zum Gebrauch üblicherweise mit einem klebstoffabweisenden Schutzabdeckung, wie silikonisiertes Papier, vorgesehen. Dieses schützt die Selbstklebeschicht aus einer gut hautverträglichen Klebemasse, beispielsweise auf Acrylatbsis, die vorzugsweise im Transferverfahren aufgebracht worden ist, und stabilisiert zusätzlich das ganze Produkt. Die Abdeckung ist zweiteilig ausgebildet. Sie ist vorzugsweise etwas breiter als das Trägermaterial und die Folie, um deren Randkanten zu schützen.

Das Verbandmaterial kann als solches verwendet werden, es kann jedoch auch zusätzlich mittig in geeigneter Breite eine übliche, saugende Wundauflage aufgebracht sein, so daß es direkt als Wundverband eingesetzt werden kann. Ein derartiger Verband mit Rundum-Verklebung ist besonders vorteilhaft, da er keimdicht und wasserfest ist.

Das erfindungsgemäße Verbandmaterial wird in Form einzelner vorgefertigter Abschnitte und evtl. durch Gamma-Strahlen sterilisiert und eingesiegelt in verschiedenen Längen und Breiten angeboten. Beim Gebrauch wird einfach nur die Schutzabdeckung über der Klebeschicht entfernt, das Material beidseitig an den Griffleisten erfaßt und bedarfsmäßig aufgeklebt. Anschließend wird die leicht wiederablösbare Trägerschicht auf der Rückseite der Folie mit den Griffleisten rückstandsfrei abgezogen.

Zur Handhabung des Verbandes bedarf es keiner Gebrauchsanleitung, da sich diese wie von selbst ergibt. Da der ganze Verband einschließlich der stützenden Trägerschicht zudem transparent ist, kann er ohne Schwierigkeiten paßgenau angebracht werden.

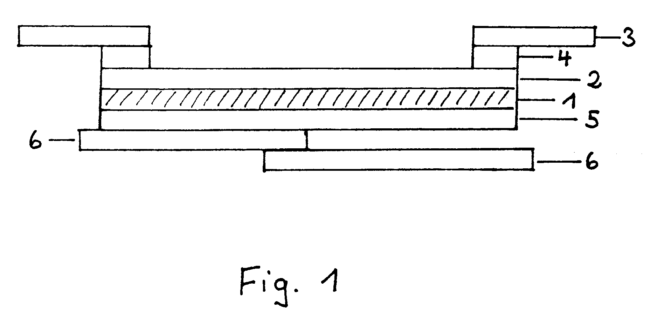

In Figur 1 ist das erfindungsgemäße Verbandmaterial mit vergrößerten Dicken der Schichten beispielsweise dargestellt. Dabei bedeuten 1 die Folie, 2 die Trägerschicht, 3 die Griffleisten, 4 eine Klebeschicht zur Befestigung der Griffleisten am Träger, 5 die Selbstklebeschicht und 6 die Schutzabdeckung für die Selbstklebeschicht.

## Patentansprüche

1. Verbandmaterial auf Folienbasis, bestehend aus einer Folie (1) die auf der einen Seite auf einer flexiblen u. anschmiegsamen Trägerschicht (2) ablösbar direkt aufgebracht ist durch Gießen, Rakeln, Extrudieren oder dergleichen übliche Methoden der Filmherstellung, wobei beide Schichten (1,2) die gleichen Größen aufweisen, u. welche Folie (1) auf der anderen Seite mit einer selbstklebenden Schicht (5) versehen ist, die ihrerseits eine klebstoffabweisende Schutzabdeckung (6) aufweist,
dadurch gekennzeichnet daß
- die Trägerschicht (2) einteilig ausgebildet ist,
- separate Griffleisten (3) entlang zweier gegenüberliegender Ränder der Trägerschicht (2) fest angebracht sind, derart, daß die Trägerschicht (2) mit Hilfe der Griffleisten (3) von der Folie (1) ablösbar ist, wobei die Griffleisten (3) nur mit einem Teil ihrer Breite mit der Trägerschicht (2) verbunden sind u. mit dem anderen, vorzugsweise größeren Teil ihrer Breite, über das Verbandmaterial (1) hinausragen,
- die Haftung der Folie (1) auf der Trägerschicht (2) zw. 0.01 u.0.5 N/cm beträgt u. damit wesentlich geringer ist als die Haftung der Klebeschicht (5) auf der Haut nach Applikation des Verbandmaterials u.
- die Schutzabdeckung (6) überlappend zweigeteilt ist.

2. Verbandmaterial gemäß Anspruch 1, dadurch gekennzeichnet, daß die Haftung zwischen der Folie (1) und dem Trägermaterial (2) 0,01 - 0,05 N/cm beträgt.

3. Verbandmaterial gemäß Anspruch 1, dadurch gekennzeichnet, daß das Trägermaterial (2) aus einer matten, transparenten Polethylenfolie besteht.

4. Verbandmaterial gemäß Anspruch 1, dadurch gekennzeichnet, daß die Griffleisten (3) aus einer LDPE-Folie bestehen.

5. Verbandmaterial gemäß Anspruch 1, dadurch gekennzeichnet, daß die Griffleisten (3) an dem Trägermaterial (2) angeklebt oder angeschweißt sind.

6. Verbandmaterial gemäß Anspruch 1, dadurch gekennzeichnet, daß die Folie (1) aus Polyurethan besteht.

7. Verbandmaterial gemäß Anspruch 1, dadurch gekennzeichnet, daß auf der klebenden Seite eine Wundauflage angeordnet ist, welche kleiner ist als die Klebefläche, so daß klebende Ränder frei bleiben.

8. Verbandmaterial gemäß Anspruch 1, dadurch gekennzeichnet, daß die Schutzabdeckung (6) etwas breiter als die Trägerschicht (2) u. die Folie ist.

## Claims

1. Film-based bandage material, comprising a film (1) applied directly on one side in a detachable manner to a flexible and pliable base layer (2) by casting, knife coating, extrusion or similar conventional methods of film production, where the two layers (1, 2) have the same sizes, and which film (1) is provided on the other side with a self-adhesive layer (5) which itself has an adhesive-repellent protective cover (6), characterized in that
- the base layer (2) is formed in one part,
- separate grip strips (3) are firmly arranged along two opposite edges of the base layer (2) in such a way that the base layer (2) can be detached from the film (1) with the aid of the grip strips (3), the grip strips (3) being connected to the base layer (2) only through part of their width and projecting beyond the bandage saterial (1) with the other, preferably larger part of their width,
- the adhesion of the film (1) to the base layer (2) is between 0.01 and 0.5 N/cm and is thus significantly lower than the adhesion of the adhesive layer (5) to the skin after application of the bandage material, and
- the protective cover (6) is formed from two overlapping parts.

2. Bandage material according to Claim 1, characterized in that the adhesion between the film (1) and the base material (2) is 0.01 - 0.05 N/cm.

3. Bandage material according to Claim 1, characterized in that the base material (2) comprises a matt, transparent polyethylene film.

4. Bandage material according to Claim 1, characterized in that the grip strips (3) comprise an LDPE film.

5. Bandage material according to Claim 1, characterized in that the grip strips (3) are adhesively bonded or welded to the base material (2).

6. Bandage material according to Claim 1, characterized in that the film (1) comprises polyurethane.

7. Bandage material according to Claim 1, characterized in that a wound pad which is smaller than the adhesive area is arranged on the adhesive side so that adhesive edges remain free.

8. Bandage material according to Claim 1, characterized in that the protective cover (6) is somewhat wider than the base layer (2) and the film.

## Revendications

1. Pansement à base de film, se composant d'un film (1) qui est appliqué de l'un des côtés directement en restant détachable, sur une couche de support (2) souple et capable d'épouser la forme du support, par coulage, par raclage, par extrusion ou par un procédé similaire, les des couches (1, 2) présentant les mêmes tailles et lequel film (1) étant pourvu de l'autre côté d'une couche auto-adhésive (5), qui, à son tour, présente un recouvrement de protection (6) auto-adhésif,
caractérisé
- en ce que la couche de support (2) est formée d'une seule pièce,
- en ce que des rebords de prise séparés (3) sont attachés de manière solide le long des deux bords opposés de la couche de support (2) de sorte que l'on peut détacher la couche de support (2) à l'aide des rebords de prise (3) du film (1), les rebords de prise (3) étant reliés seulement sur une partie de leur largeur avec la couche de support (2) et dépassant avec l'autre partie, de préférence plus grande, de leur largeur, du pansement (1),
- en ce que l'adhésion du film (1) sur la couche de support (2) est comprise entre 0,01 et 0,5 N/cm et est par conséquent substantiellement plus faible que l'adhésion de la couche adhésive (5) sur la peau après application du pansement, et
- en ce que le recouvrement de protection (6) est divisé en deux en présentant un chevauchement.

2. Pansement selon la revendication 1, caractérisé en ce que l'adhésion entre le film (1) et le matériau de support (2) est de 0,01 - 0,05 N/cm.

3. Pansement selon la revendication 1, caractérisé en ce que le matériau de support (2) se compose d'un film de polyéthylène mat, transparent.

4. Pansement selon la revendication 1, caractérisé en ce que les rebords de prise (3) se composent d'un film en polyéthylène de faible densité LDPE.

5. Pansement selon la revendication 1, caractérisé en ce que les rebords de prise (3) sont collées ou sont soudées au matériau de support (2).

6. Pansement selon la revendication 1, caractérisé en ce que le film (1) se compose de polyuréthane.

7. Pansement selon la revendication 1, caractérisé en ce que, sur le côté adhésif, est disposé une compresse pour la blessure proprement dite, qui est plus petite que la zone adhésive, de sorte que des bords adhésifs subsistent.

8. Pansement selon la revendication 1, caractérisé en ce que le recouvrement de protection (6) est quelque peu plus large que la couche de support (2) et que le film.
